# EUROPÄISCHE PATENTSCHRIFT

(11) **EP 2 866 881 B1**
(45) Veröffentlichungstag und Bekanntmachung des Hinweises auf die Patenterteilung: **13.12.2017**
(21) Anmeldenummer: 13765283.0
(22) Anmeldetag: 28.06.2013
(51) Int. Cl.: A61N 1/04, A61N 1/36

(54) **VORRICHTUNG ZUR MUSKULÄREN STIMULATION**
DEVICE FOR MUSCLE STIMULATION
DISPOSITIF DE STIMULATION MUSCULAIRE

(30) Priorität: 28.06.2012 DE 202012102393 U; 21.01.2013 DE 102013100586
(43) Veröffentlichungstag der Anmeldung: 06.05.2015
(73) Patentinhaber: Fritzsche, Dirk, 04155 Leipzig (DE)
(72) Erfinder: Fritzsche, Dirk, 04155 Leipzig (DE)
(74) Vertreter: Gottfried, Hans-Peter
(86) Internationale Anmeldenummer: PCT/DE2013/100235
(87) Internationale Veröffentlichungsnummer: WO 2014/000736

(56) Entgegenhaltungen:
- WO-A1-2004/006700
- WO-A1-2004/058346
- WO-A1-2005/079910
- WO-A2-01/02052
- DE-U1-202008 017 860
- US-A1- 2005 131 489

## Beschreibung

Die Erfindung betrifft eine Vorrichtung zur muskulären Stimulation von Rumpf- und/ oder Beinmuskulatur durch Elektromyostimulation, umfassend einen wenigstens eine integrierte textilbasierte Kontaktelektrode und wenigstens eine elektrische Leitung aufweisenden, an einem Körper anliegenden, ein- oder mehrteiligen, ein elastisches textiles Flächengebilde umfassenden Anzug, der als wenigstens ein Flachgestrick ausgeführt ist, wobei die Kontaktelektrode als ein zumindest einschichtiges, als Intarsie ausgebildetes Flachgestrick zumindest teilweise aus elektrisch leitfähigem Faden ausgeführt ist.

Elektrische Muskelstimulation hat sich von einem Verfahren für spezielle Indikationen im Hochleistungssport über standardisierte Techniken in der medizinischen Rehabilitation hin zu einem sich rasch verbreitenden Verfahren in den Bereichen Fitness und "medical Fitness" entwickelt. Die Wirkungen auf das Endorgan Muskulatur und auf den Gesamtorganismus sind an zahlreichen Stellen beschrieben und wissenschaftlich validiert. Es gibt aber bislang kaum Applikationen, die eine Nutzerfreundlichkeit und Bedienfreundlichkeit aufweisen, welche es den Zielgruppen es ermöglicht, die Methode in einem häuslichen Umfeld und ohne fremde Hilfe nutzen zu können.

Eine bekannte Vorrichtung zum Muskeltraining durch Elektrostimulation ist aus der Druckschrift DE 102 48 235 A1 bekannt, wobei gleichzeitig eine Vielzahl von Körperpartien, insbesondere die komplette Bauch- und Rückenmuskulatur, Schulter-, Brust-, Atmungs-, Arm-, Bein- und Gesäßmuskulatur sowie der Beckenboden inklusive aller tiefer gelegenen Muskelgruppen stimuliert werden. Dies wird erreicht, indem jeweils eine Mehrzahl von Elektroden am Körper befestigt wird, die an einem vorzugsweise über einem Bekleidungsstück zu tragenden Isolationsträger angeordnet sind. Ein solcher Isolationsträger kann bevorzugt einen Brustgurt und/ oder einen Bauchgurt umfassen, die zur gleichzeitigen Verwendung miteinander verbindbar sind.

Vergleichbare Vorrichtungen zeigen die Druckschriften US 2002/0133195 A1, AT 401 731 B und DE 202 00 685 U1.

Nachteilig an diesen Vorrichtungen zum Muskeltraining ist, dass das Anlegen relativ umständlich ist und großer Sorgfalt bedarf, damit alle Elektroden genau auf den gewünschten Körperregionen zur Anlage kommen. Ein weiterer Nachteil besteht im aufwändigen Verkabeln der Elektroden. Die Elektroden können verrutschen, Anschlüsse können sich lösen, und es können aufgrund konkaver Körperregionen Bereiche entstehen, innerhalb derer die Elektroden keinen vollständigen Körperkontakt haben. Schließlich ist auf eine genügend wirksame Durchfeuchtung der Unterkleidung zu achten, wobei ein Training mit durchfeuchteter Kleidung als unangenehm empfunden werden kann. Des Weiteren ist die umfangreiche Verkabelung zwischen dem Trainierenden und dem Stimulationsgerät teilweise bewegungseinschränkend und unkomfortabel.

Weitere Vorrichtungen zur elektrischen Muskelstimulation, beispielsweise nach der Druckschrift EP 0 459 945 B1, sehen vor, dass kreisförmige bzw. annähernd kreisförmige Elektroden direkt auf der Haut platziert werden. Dies erfordert genaue Anweisungen, beispielsweise eines Trainers oder Therapeuten. Im medizinischen und anderen Bereichen werden insbesondere Kunststoff-Klebeelektroden eingesetzt, die von erfahrenem Fachpersonal am Körper lokalisiert werden müssen und zugleich unangenehm im Hautkontakt sind.

Die Druckschrift US 2007/0049814 A1 offenbart ein Komplettsystem, bestehend aus einem mit Elektroden versehenen Anzug, wobei die Elektroden mit einem programmierbaren Stimulatorgerät verbunden sind. Die Verbindung kann dabei per Leitung, jedoch auch drahtlos erfolgen. Bei einer drahtlosen Verbindung erscheint die Versorgung der Elektroden mit Elektroenergie problematisch, währenddessen ein leitungsgebundenes Gerät die Bewegungsfreiheit des Nutzers einschränkt und Unfallgefahren birgt, zumal wenn so wie vorgesehen Steckverbinder verwendet werden. Zudem erfolgt die Bedienung des Elektromyostimulation über das Stimulatorgerät in der Weise, dass Programme abgerufen werden. Diese Programme schränken die Flexibilität der Ansteuerung ein, so dass eine individuelle bzw. akut angepasste Betriebsweise nicht möglich wird.

Die Druckschrift WO 2004/006700 A1 beschreibt eine Vorrichtung zur Elektromyostimulation, die jedoch v.a. der Signalgewinnung vom Körper (aber auch der Signaleinleitung) dient. Die Elektroden sind in ein Kleidungsstück eingestrickt, ebenso die Zuleitungen (quer durch das Trägermaterial und auch längs). Die Elektroden sind unter Verwendung leitfähiger, z.B. silberhaltiger Fasern, während eines Rundstrickvorgangs eingestrickt (oberflächlich oder durchgehend).
Vorgesehen sind nach der Druckschrift WO 2004/006700 A1 auch mehrschichtige Gestricke (durch Zusammenschlagen eines spiegelbildlichen Doppelgestricks oder Übereinanderstricken von zwei Kleidungsstücken), wodurch sich weitere Möglichkeiten zur Anordnung von Elektroden und Leitungen ergeben.
Allerdings ist ein Flachstricken des Anzugs bzw. des textilen Trägers der Elektroden nicht vorgesehen, so dass die entsprechenden Eigenschaften nicht erzielbar sind.

Nach der Druckschrift US 2007/0049814 A1 ist ein transkutanes neuromuskuläres Stimulationssystem bekannt, bei dem eine flexible, flache Elektrode an einem Anzug aufgebracht oder eingebracht ist. Ein programmierbares elektrisches Stimulationsgerät ist mit den Elektroden verbindbar. Die Elektroden weisen eine erste Gewebeschicht auf, ein Stück silberbehandeltes Material (mit Silber beschichtet oder aus silberbeschichteten Fasern gewebt), das in Kontakt mit der Gewebeschicht steht, ein Stück teilweise abisolierten elektrischen Draht, der in Kontakt mit dem Silber steht. Weiterhin ist eine zweite Gewebeschicht vorhanden, die die erste abdeckt, und ein Kontakt zum elektrischen Anschluss.
Die Elektroden können zum Schutz oder anderweitig zur Verlängerung der Lebensdauer beschichtet sein, wozu ein elektrisch leitfähiges, weitgehend festes oder flüssiges Gel in Verbindung mit der Elektrode (entweder direkt auf das Gewebe, in Kontakt mit der Haut, oder in einem Gelreservoir) steht. Jede Elektrode besitzt ihren Leiter als Zuleitung.
Ein Gel als Beschichtung hat eine geringe Haltbarkeit und Lebensdauer, da es leicht beschädigt werden kann, sich von der Oberfläche ablöst oder austrocknet. Zudem ist eine feste und sichere Verbindung mit dem Untergrund nicht möglich. Weiterhin ist ein Flachstricken des Anzugs bzw. der Gewebeschicht nicht vorgesehen, so dass die entsprechenden Eigenschaften nicht erzielbar sind.

Nach dem Gebrauchsmuster DE 20 2011 109 226 U1 ist die Energieversorgung für eine Elektromyostimulationsvorrichtung zwar im Anzug integriert, die Steuerimpulse kommen jedoch von außerhalb, von einem Steuergerät, das mit dem Anzug verbunden wird. Die Zuleitungen zu den Elektroden sind als leitfähiges Nähgarn (z.B. silberbeschichtetes PA mit isolierender Ummantelung) ausgeführt oder in das Trägermaterial auf- oder eingenäht oder -gestickt, alternativ aber auch zwischen Lagen der Trägerschicht eingestrickt, eingewirkt oder eingewebt. Die Kontaktelemente am Anzug sind als Druckknöpfe ausgeführt. Die Trockenelektrode besteht aus Silbergewebe, das auf das Neoprenträgermaterial (oder Lycra, Elasthan) aufkaschiert (geplottet, kaschiert) ist. Eine solche oberflächliche Kaschierung kann sich bei Gebrauch lösen, insbesondere wenn das darunterliegende Gewebe gedehnt wird. Zudem ist ein Flachstricken der textilen Unterlage der Elektroden nicht vorgesehen, so dass die entsprechenden Eigenschaften nicht erzielbar sind.

Die Druckschrift DE 20 2008 017 860 U1 beschreibt einen modifizierten Kompressionsstrumpf zur Durchführung der Elektromyostimulation, wobei die Elektroden am Kompressionskörper angebracht sind. Die Elektroden sind flach bzw. haben geringen Überstand, weisen eine Fläche von 5 oder 10 cm² auf und bestehen aus elektrisch gut leitendem, elastischem Material. Eine Steuereinheit ist am Kompressionskörper vorgesehen, alternativ auch eine Fernsteuerung. Die Anbindung erfolgt durch Kabel oder drahtlos. Allerdings ist ein Flachstricken der textilen Unterlage der Elektroden nicht vorgesehen, so dass die entsprechenden Eigenschaften nicht erzielbar sind.

In der Druckschrift DE 10 2006 058 346 A1 ist eine Elektromyostimulationsvorrichtung mit Bewegungssensor beschrieben, wobei die Elektromyostimulation von der Bewegung abhängig ist und die Vorrichtung Fehlbewegungen entgegenwirkt. Die Elektrodenfelder sind mit einzelnen, ansteuerbaren Elektroden zur exakten Positionierung der Einleitung ausgestattet. Eine Speicheranordnung für Bewegungsmuster ist vorgesehen, unterschieden werden Steuereinrichtung, Stimulationseinrichtung und Elektrodensteuereinrichtung. Eine Signalverbindungseinrichtung zwischen Steuereinrichtung und Stimulationseinrichtung ist vorgesehen. Allerdings werden der Anzug bzw. die textile Unterlage der Elektroden nicht durch Flachstricken erzeugt, so dass die entsprechenden Eigenschaften nicht erzielbar sind.

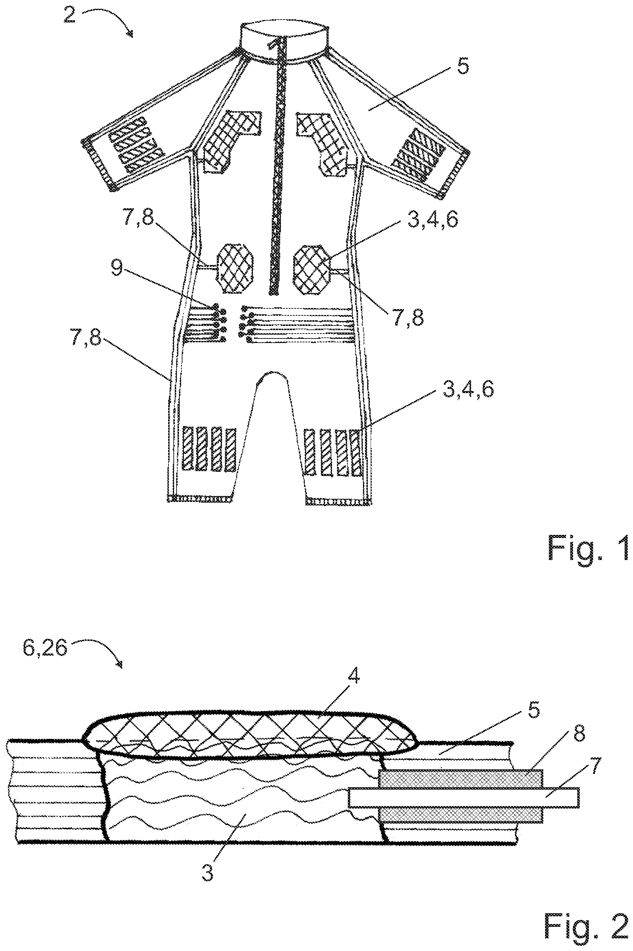

Die Druckschrift WO 01/02052 A2 offenbart eine Vorrichtung (Fig. 1, 6) zur muskulären Stimulation von Rumpf- und/oder Beinmuskulatur durch Elektromyostimulation, umfassend ein wenigstens eine integrierte textilbasierte Kontaktelektrode (Fig. 1, Bz. 11) aufweisendes, an einem Körper anliegendes, ein- oder mehrteiliges, elastisches, gestricktes Kleidungsstück, wobei die Kontaktelektrode als ein zumindest einschichtiges, als Intarsie ausgebildetes Gestrick zumindest teilweise aus elektrisch leitfähigen Fäden ausgebildet ist. Nachteilig an dieser Lösung ist jedoch, dass an den Kontaktstellen zwischen der Haut und einem leitfähigen Faden regelmäßig lokal hohe Ströme fließen, die zu unangenehmen Empfindungen wie Stechen führen. Zur Vermeidung dessen schlägt die Druckschrift vor, zusätzlich ein Gel aufzutragen. Solche Gels sind bekannt und werden allgemein genutzt in der Elektrotherapie und Physiotherapie, wie z. B. Dermedics EEG als ein hoch leitfähiges medizinisches Kontaktgel. Dies ist aber mit zusätzlichem Aufwand verbunden und erfordert nachträglich eine Reinigung von Körper und Kleidungsstück von dem Gel.

Die DE 600 38 341 T2 beschreibt eine Muskelstimulation in Abhängigkeit von der Herzfrequenz und anderer physiologischer Werte, wozu diese Werte durch Sensorelektroden erfasst und in einer Steuereinrichtung in verschiedenen komplizierten Algorhythmen verarbeitet werden. Sowohl die Sensorelektroden als auch die Elektroden zum Aufbringen der Stimulationsimpulse, die aktiven Elektroden, sind jedoch Elektroden nach dem Stand der Technik, darunter auch Akkupunkturelektroden oder implantierte Elektroden. Als Elektrodenträger sind Gurte oder Sitze vorgesehen. Eine komfortable und einfache Nutzungsmöglichkeit ist nicht gegeben. Zudem ist ein Flachstricken des Anzugs bzw. der textilen Unterlage der Elektroden nicht vorgesehen, so dass die entsprechenden Eigenschaften nicht erzielbar sind.

Bei einem Rundgestrick hingegen wäre lediglich eine Verjüngung oder Erweiterung des Gestrickschlauchs möglich. Wesentlich ist die partielle, dreidimensionale Ausformung des Gestricks mit konturengenauer Abbildung und Anpassung vor allem der Intarsien- bzw. Elektrodenformen an Körper- und Muskelform, was in dieser Weise ausschließlich mit der Flachstricktechnologie realisierbar ist.

Auch andere Verfahren einer Elektromyostimulation, wie zum Beispiel Miha-Bodytec.de, Amplitrain, sind ohne Hilfsperson nicht durchführbar und damit nicht geeignet, in einem häuslichen Umfeld von einem Patienten allein angewandt zu werden. Gründe sind beispielsweise eine aufwändige Verkabelung oder komplizierte Einstellungen an der Bedieneinheit.

Die Erfindung hat die Aufgabe, eine Vorrichtung anzubieten, mit deren Hilfe der unumstrittene Nutzen der Methode der Elektromyostimulation für Patienten in ihrem häuslichen Umfeld nutzbar gemacht werden kann. Weiterhin ist vorgesehen, die Akzeptanz zu verbessern und Anwendungsprobleme bei der Elektromyostimulation zu vermeiden. Es soll eine geeignete Vorrichtung zur muskulären Stimulation von Rumpf-und Beinmuskulatur angeboten werden, die flexibel einsetzbar sowie sicher, komfortabel und einfach zu bedienen ist.

Die Aufgabe der Erfindung wird gelöst durch eine Vorrichtung zur muskulären Stimulation von Rumpf- und/ oder Beinmuskulatur durch Elektromyostimulation, umfassend einen wenigstens eine integrierte textilbasierte Kontaktelektrode und wenigstens eine elektrische Leitung aufweisenden, an einem Körper anliegenden, ein- oder mehrteiligen, aus einem elastischen textilen Flächengebilde bestehenden Anzug. Das textile Flächengebilde ist dabei als Flachgestrick ausgeführt, besonders bevorzugt als einflächiges Flachgestrick ohne Nähte und ohne das Erfordernis, Teile zusammenzunähen. Durch das Flachgestrick ist eine dreidimensionale Angleichung des Gestricks, beispielsweise mittels Anwendung verschiedener, auf Intarsienflächen bezogener Strickbindungen, an die Konturen des Körpers möglich, so dass der Anzug stets vollflächig körpernah anliegt. Das dreidimensionale Flachgestrick ermöglicht im Gegensatz zum Rundgestrick eine partielle Kompression und eine optimale Anpassung an die Formen des Körpers.

Zudem ist in dem textilen Flächengebilde wenigstens eine elektrische Leitung in einer gestrickten Tunnelröhre angeordnet, mittels derer die Kontaktelektroden elektrisch konnektierbar sind. Diese Leitungen sind bevorzugt textilbasiert, also mittels textiltechnischem Verfahren unter Verwendung elektrisch leitfähiger Fäden hergestellt, besonders bevorzugt gestrickt. Das Stricken kann gesondert erfolgen mit dem Ziel, die so erhaltenen Leitungen in die Tunnelröhren einzubringen. Ebenso ist es jedoch vorgesehen, das Flachgestrick des Anzugs, die Tunnelröhren und die Leitungen in einem Arbeitsgang zu stricken.

Es sind jedoch auch andere Arten von Leitungen, z.B. metallische Drähte oder Litzen, vorgesehen. Diese Leitungen sind bei einer bevorzugten Ausgestaltung in einem rundum geschlossenen, als Tunnelröhre gestrickten Rundkanal angeordnet, besonders bevorzugt eingelegt, alternativ separat gestrickt oder in anderer Weise eingearbeitet. Die Tunnelröhre ist bevorzugt als flachgestrickte Tunnelröhre ausgebildet. Die Leitungen liegen somit direkt im Gestrick vor. Die Flächen mit den eingefügten elektrischen Leitern werden danach in der Weise verbunden, dass ein elektrischer Kontakt über die einzelnen flachgestrickten Flächen hinweg erfolgt. Damit sind die Kontaktelektroden elektrisch kontaktierbar und eine elektrische Verbindung zwischen einer Kontaktstelle am Anzug, wo das Stimulationssignal aus der Stimulationseinheit eingespeist wird, und der Kontaktelektrode wird möglich. Eine Kontaktierung der elektrischen Leiter miteinander ist vor allem dann erforderlich, wenn der Anzug aus mehreren Flächen von Flachgestrick besteht, die miteinander verbunden werden müssen, z.B. durch Vernähen. Die elektrischen Leitungen werden beim Zusammenfügen zum Anzug zur elektrisch leitenden Verbindung kontaktiert, beispielsweise vernäht, verklebt oder geklemmt, wobei jeweils die elektrische Verbindung erhalten bleibt. Entsprechend erfolgt die Verbindung zwischen Leiter und Kontaktelektrode.

Weiterhin sind eine oder mehrere Kontaktelektroden als ein zumindest einschichtiges, als Intarsie ausgebildetes Flachgestrick, das durch Verwendung elektrisch leitfähiger Fäden zumindest teilweise aus elektrisch leitfähigem Material besteht, und mit einer körperseitigen elektrisch leitfähigen Stimulationsschicht ausgebildet. Als Intarsie wird hier eine ebenfalls als Flachgestrick erzeugte Fläche innerhalb des den Anzug bildenden Flachgestricks bezeichnet, zu deren Herstellung zumindest teilweise elektrisch leitfähige Fäden, z.B. elektrisch leitfähiges Garn zum Einsatz kommen. Deren Leitfähigkeit kann sowohl auf chemischen Effekten (wie z.B. Kohlenstoffpolymere) als auch auf physikalischen Effekten (wie Metalle, Metalllegierungen) sowie deren Kombinationen (z.B. metallische Synthetikbeschichtungen) beruhen. Der leitfähige Faden kommt ebenfalls bei der Herstellung der elektrischen Leitung zum Einsatz. Die Intarsie kann an einer oder beiden Oberflächen des umgebenden Flachgestricks, alternativ auch eines anderen Gestricks oder Gewirkes angearbeitet sein und in diesen eindringen. Alternativ kann die Intarsie in dem vollem Querschnitt des umgebenden Flachgestricks vorliegen und diesen komplett durchdringen.

Insbesondere ist die Kontaktelektrode in einer bevorzugten Ausgestaltung der Erfindung aber zumindest an der dem Körper zugewandten Seite mit einer elastischen, elektrisch leitfähigen Implantierung als Stimulationsschicht mit Hautberührung ausgestattet. Diese bedeutet, dass nicht nur eine Beschichtung vorhanden ist, sondern sich das Implantierungsmaterial sowohl an der Oberfläche der Intarsie der Kontaktelektrode, als auch zumindest teilweise in deren Inneres erstreckt, z.B. zwischen die Maschen eines Flachgestricks wandert. Dadurch werden eine sehr hohe Haltbarkeit und auch ein besonders sicherer elektrischer Kontakt erreicht. Dehnungen des Flachgestricks werden schonend auf die Implantierung übertragen, so dass eine Ablösung vom Gestrick dauerhaft vermieden und zudem ein hoher Tragekomfort gewährleistet werden. Die Implantierung über deckt die Intarsie zumindest teilweise, kann aber auch über die Intarsie hinaus sich bis auf das Flachgestrick hin erstrecken. Bevorzugt wird zur Implantierung ein Polymer verwendet, wobei die Implantierung beispielsweise mittels Pressdruck, Wärme oder ähnlichen geeigneten Verfahren erfolgt. Durch den Einsatz eines Polymers, bevorzugt eines Kautschuks, wird beim Einsatz eine passive Hydrierung durch lokale Feuchtigkeitsentwicklung an der Haut, somit durch Nutzung der Körperfeuchtigkeit, bewirkt. Die Elektroden benötigen aus diesem Grund keine zusätzliche Anregung durch Feuchtekissen und auch keine Erhöhung der elektrischen Leitfähigkeit mittels eines Gels oder Gelreservoirs.

Die ein weiches Kissen ausbildende Beschichtung liegt angenehm auf der Haut und verteilt durch ihre elektrischen Eigenschaften den Strom sehr gleichmäßig über die gesamte an der Haut anliegende Fläche. Dadurch kommt es nicht zu unangenehmen lokalen Stromspitzen; der Stromeintritt in die Haut bleibt für den Nutzer angenehm. Die elektrisch leitfähige Beschichtung stellt nur ein Zusatzelement der Elektrode dar, die bereits fertig und funktionsfähig aus dem textiltechnischen Prozess hervorgeht und bereits geeignete elektrische Eigenschaften aufweist. Besonders günstige Trageeigenschaften und die vorgenannten Vorteile werden jedoch erreicht, wenn die Beschichtung aus einer elektrisch leitfähigen Dispersion oder einem elektrisch leitfähigen Polymer besteht, da diese Materialien weich an der Haut anliegen.

Als Material für die Kontaktelektroden und/ oder deren Stimulationsschicht kommen elektrisch leitfähige Polymermassen in Betracht, die an den textilen Strukturen angeordnet sind und in diese eindringen. Bevorzugt ist ein elektrisch leitender Kautschuk vorgesehen, der besonders bevorzugt textilbasierte Elektroden auf dem textilen Flächengebilde ausbildet, das bevorzugt hierbei als ein Flachgestrick ausgeführt ist. Es kommen jedoch auch andere Arten eines textilen Flächengebildes, z.B. Gewirke oder Rundgestricke, sowie anderen Arten der elektrischen Leitung, wie beispielsweise isolierte Litzen, in Betracht.

Weitere Vorteile der erfindungsgemäßen Vorrichtung bestehen darin, dass im Flachgestrick nur eine einzige Gestrickfläche vorliegt, diese aber in verschiedensten Strickbindungen je nach den benötigten dreidimensionalen Konturen ausführbar ist. Dabei wird eine Ausformungen entsprechend der Formen von Körper und Muskel ermöglicht, wodurch die Elektrode konturengenau die Muskel- und Körpergeometrie abzubilden in der Lage und damit eine optimale Übereinstimmung und Anlage der Elektrode an der Haut realisierbar ist. Weiterhin wird der elektrische Leiter zum elektrischen Anschluss der Kontaktelektrode mittels eines eingestrickten Tunnels umschlossen, er ist damit isoliert, geschützt und nicht sichtbar. Der Tunnel oder die Tunnelröhre wird bevorzugt zusammen mit dem Flachgestrick, der Tunnelröhre und der elektrischen Leitung miteinander ausgebildet, beispielsweise in einem Arbeitsgang gestrickt. Alternativ ist es jedoch auch vorgesehen, die elektrische Leitung nachträglich in den Tunnel einzubringen, vor allem, wenn es sich nicht um eine gestrickte Leitung handelt, sondern eine Litze oder ähnliches.

Zudem weist die Kontaktelektrode ein implantiertes Polymer auf, das eine passive Hydrierung der Hautoberfläche in Richtung hin zur Gestrickfläche bewirkt und wodurch eine optimale elektrische Leitfähigkeit durch geringen Übergangswiderstand der Elektrodenoberfläche zum Körper gesichert und gleichzeitig der Tragekomfort des Kleidungsteils wesentlich verbessert wird. Darüber hinaus führt die konsequente Herstellung aller textilen Elemente im Verfahren des Flachstrickens zu einer hohen Effektivität bei der Herstellung der erfindungsgemäßen Vorrichtung zur muskulären Stimulation durch Elektromyostimulation.

Als Anzug ist jedes Kleidungsstück anzusehen, dass die erfindungsgemäßen Merkmale und Funktionen aufweist. Es kann der komplette Anzug zum Einsatz kommen, um Rumpf- und Beinmuskulatur zu stimulieren, oder einzelne Teile wie ein Oberteil oder eine Strumpfhose, um einzelne Bereiche des Körpers, beispielsweise den Rumpf oder die Beine, einzeln stimulieren zu können.

Die Kontaktelektroden sind mit Hilfe des Anzugs an vorgesehenen Stellen am Körper positioniert, um dort stimulierende elektrische Impulse einzuleiten. Hierzu sind die Kontaktelektroden mit der Stimulationseinheit elektrisch leitend verbunden.

Die Bedienung erfolgt jedoch bevorzugt nicht direkt über die Stimulationseinheit, wobei eine solche Lösung von der Erfindung ebenfalls umfasst ist, sondern bevorzugt in komfortabler Weise über eine Bedieneinheit. Die Signale, von der Bedieneinheit bei der Eingabe von Bedienbefehlen erzeugt, werden über die Kommunikationseinheit auf die Stimulationseinheit übertragen. Hierdurch ermöglicht die erfindungsgemäße Vorrichtung eine zugleich einfache und flexible Bedienung durch den Nutzer, der damit Art und Stärke der Stimulation seiner Muskeln umfassend beeinflussen, vorgegebene Programme nutzen oder die Stimulation individuell einstellen kann. Hierdurch wird der Wunsch erfüllt, ähnlich positive, gesundheitsfördernde Effekte wie mit einem regelmäßigen konventionellen Training, jedoch mit geringerer Zeitinvestition und möglichst kurzfristig realisieren zu können.

Vorteilhaft ist ein Anzug, der am Körper anliegt und ein- oder mehrteilig aus einem elastischen textilen Flächengebilde mit integrierten textilbasierten Kontaktelektroden und in dem textilen Flächengebilde integrierten, textilbasierten elektrischen Leitungen ausgebildet ist. Ein am Körper anliegender, elastischer Anzug erhöht das Wohlbefinden und hält den Bediener warm als optimale Voraussetzungen für ein erfolgreiches Training. Entsprechend den Vorlieben des Nutzers oder den Umständen, unter denen die Vorrichtung benutzt wird, kann der Anzug einteilig sein.

Besonders vorteilhaft ist es, wenn die elektrischen Leitungen textilbasiert ausgebildet und damit, ohne die Elastizität oder andere Trageeigenschaften des Anzugs zu beeinträchtigen, in das textile Flächengebilde funktionell in jeder Hinsicht integriert sind. Dies kann beispielsweise durch metallbeschichtete, bevorzugt isolierte Kunststofffasern erfolgen, die zusammen mit den anderen das textile Flächengebilde bildenden Fasern verarbeitet werden. Damit können die Signale von der Stimulationseinheit durch das textile Flächengebilde, das den Anzug bildet, zu den Kontaktelektroden geleitet werden.

Besonders günstig ist auch eine Strumpfhose mit wenigstens einer zirkumferentiellen Kontaktelektrode im Bereich von Ober- oder Unterschenkel, bevorzugt ist an jedem Ober- und Unterschenkel jeweils eine Kontaktelektrode angeordnet. Dadurch können Beinmuskeln gezielt und intensiv trainiert werden, ohne einen kompletten Anzug tragen zu müssen.

Alternativ ergänzend sind die druckelastischen, textilen Abstandsstrukturen und/ oder der druckelastische, senkrechte Fadenbesatz als Feuchtespeicherkern ausgebildet sind. Hierdurch wird eine besonders gute elektrische Leitfähigkeit hervorgerufen. Alternative Ausführungsformen zeichnen sich wie vorstehend beschrieben durch eine Kontaktelektrode aus, die aufgrund der Materialzusammensetzung auf eine Befeuchtung nicht angewiesen ist.

Bevorzugt werden sämtliche Kontaktelektroden, zumindest jedoch ein Teil davon, von einer Stimulationseinheit als steuerbarer Energieversorgung mit Stimulationsstrom versorgt. Die mehrteilige, bevorzugt zweiteilige Variante des Anzugs der erfindungsgemäßen Vorrichtung ist so gestaltet, das jeder Teil eine eigene Stimulationseinheit aufweist. Alternativ hierzu weisen die Teile des Anzuges elektrische Verbindungen zumindest zu dem Teil auf, der die Stimulationseinheit umfasst. Zur Einstellung der Funktionen der Stimulationseinheit dient eine Bedieneinheit.

Besondere Vorteile resultieren, wenn die Stimulationseinheit abnehmbar mit dem Anzug verbunden ist. Damit kann der Anzug auch ohne Beeinträchtigung getragen werden, wenn kein Stimulationstraining durchgeführt wird. Zudem kann eine Stimulationseinheit von mehreren Benutzern, von denen jeder über einen individuellen Anzug verfügt, genutzt werden.

Hierbei hat es sich als günstig erwiesen, wenn zur Verbindung der Stimulationseinheit mit dem Anzug wenigstens ein metallener oder metallisierter Druckknopf vorgesehen ist. Eine solche Verbindung stellt eine gleichermaßen haltbare und einfache wie elektrisch sichere Verbindung dar. Besonders bevorzugt sind mehrere Kontakte vorgesehen. Durch die Druckknöpfe wird die Stimulationseinheit elektrisch kontaktiert und gleichermaßen fest an dem Anzug gehalten.

Alternativ oder ergänzend zum Einsatz von Druckknöpfen ist zur Verbindung wenigstens ein bevorzugt metallisiertes Klettverbindungselement vorgesehen. Eine Klettverbindung ist einfach aufgebaut und in der Weise flexibel einsetzbar, dass sie an verschiedenen Orten anheftbar ist. Wenn die Stimulationseinheit beispielsweise die Hakenseite aufweist, kann sie auf einer größeren Fläche auf dem Anzug, die mit der Flauschseite ausgestattet ist, befestigt werden. Dies kann beispielsweise so erfolgen, dass die Kontakte als offene Leiterbahnen vertikal am Anzug verlaufen und somit auch die Stimulationseinheit zur Erhöhung der Bequemlichkeit des Nutzers vertikal an unterschiedlichen Stellen angeheftet werden kann.

Als weitere Alternative ist zur Verbindung wenigstens ein Magnetkontakt vorgesehen. Magnetkontakte bieten eine sichere elektrische Verbindung und sind dennoch im Bedarfsfall schnell und leicht lösbar, beispielsweise wenn der Nutzer bei der Bewegung mit der Stimulationseinheit an ein Hindernis stößt. Dadurch werden Unfälle und eine Beschädigung des Anzuges vermieden.

Bevorzugt weist die Stimulationseinheit zur Energieversorgung einen Akkumulator auf. Der Akkumulator liefert die Energie, den elektrischen Strom zum Betrieb der Kontaktelektroden, die den Strom in den Körper einleiten. Durch einen Akkumulator, der fest installiert oder auswechselbar mit der Stimulationseinheit verbunden ist, kann die Energie schnell wieder aufgeladen werden und die Stimulationseinheit ist erneut einsatzbereit. Nach der Erfindung ist es jedoch auch vorgesehen, anstelle des Akkumulators nicht wieder aufladbare Batterien oder andere Stromquellen zu benutzen, wie beispielsweise einen Generator, diesen bevorzugt in Verbindung mit einem zumindest kurzzeitig wirksamen Speicher.

Besondere Vorteile ergeben sich, wenn die Bedieneinheit als grafische Nutzerschnittstelle, aufweisend einen berührungsempfindlichen Bildschirm, zur Steuerung der Aktivität der Kontaktelektroden ausgebildet und die Bedieneinheit von dem Anzug beabstandet ist. Durch eine beabstandete Bedieneinheit wird erreicht, dass der Anzug und die an ihm angeordneten Elemente ein minimales Gewicht aufweisen und trotzdem eine optimale Bedienung gewährleistet ist, die insbesondere auf den jeweiligen Nutzerkreis, beispielsweise ältere Menschen oder Menschen mit eingeschränkter Mobilität, ausgerichtet werden kann, ohne die Stimulationseinheit selbst verändern zu müssen.

Bei einer bevorzugten Ausführungsform sind in der Bedieneinheit abrufbare Programme zur Steuerung der Aktivität der Kontaktelektroden bei der Abgabe elektrischer Impulse abgespeichert. Damit kann die Stimulationseinheit kleiner und leichter aufgebaut und die informationstechnische Leistungsfähigkeit des System Elektromyostimulation ganz oder zum überwiegenden Teil auf die Bedieneinheit übertragen werden. Bevorzugt ist diese beabstandet zum Anzug und zur Stimulationseinheit angeordnet und wird nicht vom Nutzer getragen, während er das Stimulationstraining absolviert.

Die hohe Leistungsfähigkeit hinsichtlich der ausführbaren Programme, die die Bedieneinheit bietet, ermöglicht in einer alternativen Ausführungsform auch eine Verarbeitung automatisch generierter Eingangsdaten. Im Falle einer bidirektionalen Kommunikation zwischen Stimulations- und Bedieneinheit können beispielsweise mittels Sensoren im Anzug ermittelte Werte wie Körpertemperatur, Hautwiderstand oder physiologische Ströme herangezogen werden, um das ausgeführte Programm in seinem Ablauf in Echtzeit zu modifizieren und zu einem weiter optimierten und individualisierten Trainingsablauf zu gelangen.

Alternativ oder ergänzend hierzu ist die Aktivität wenigstens einer einzelnen Kontaktelektrode gesondert steuerbar. Ungeachtet der vielfältigen programmtechnischen Möglichkeiten, die mit der Bedieneinheit angeboten werden, soll auch eine individuelle, manuelle Ansteuerung der Kontaktelektroden möglich sein. Hierdurch kann der Nutzer schnell auf individuelle Bedürfnisse oder Wünsche reagieren, ohne ein geeignetes Programm aufrufen oder ein solches gar erstellen zu müssen.

Besondere Vorteile zeigen sich, wenn die Kommunikationseinheit zur drahtlosen Signalübertragung ausgebildet ist und eine feste Kabelverbindung zwischen Stimulationseinheit und Bedieneinheit entfallen kann. Hierdurch werden die Bewegungsfreiheit des Nutzers erhöht und Unfälle, beispielsweise durch Stolpern oder Hängenbleiben mit dem Kabel, vermieden. Zudem kann der Nutzer ungehindert Freizeitaktivitäten oder anderen Tätigkeiten nachgehen, während er das Training absolviert.

Als sehr vorteilhaft hat sich erwiesen, wenn die Kommunikationseinheit zur Signalübertragung nach Bluetooth-Standard ausgebildet ist. Da es sich um eine etablierte Kommunikationstechnik handelt, sind die entsprechenden Komponenten funktionssicher, leicht verfügbar und preisgünstig. Zudem handelt es sich um einen Übertragungsstandard, der mit sehr geringen Leistungen auskommt, so dass die unerwünschte Immission von elektromagnetischer Strahlung trotz der körpernahen Position minimal ist. Die Bluetooth-Übertragung nach Klasse 3 mit 10 m Reichweite hat eine maximale Sendeleistung von 1 mW gegenüber 2000 mW bei Mobiltelefonen zum Vergleich.

Vorteile ergeben sich aus einer Kommunikationseinheit, die zur monodirektionalen Signalübertragung der Steuersignale von der Bedieneinheit an die Stimulationseinheit ausgebildet ist. Zwar bietet die bidirektionale Übertragung, wie oben beschrieben, erweiterte Möglichkeiten zur Beeinflussung des Stimulationsalgorithmus unter Berücksichtigung physiologische Reaktionen. Eine monodirektionale (bzw. bei Bluetooth asymmetrische) Kommunikationseinheit ist jedoch einfach, kostengünstig und weist eine lange Akkulaufzeit auf, da der in der Stimulationseinheit angeordnete Teil nur als Empfänger ausgeführt ist.

Eine besonders vorteilhafte und kostengünstige Kombination von Bedieneinheit und Kommunikationseinheit erfolgt in der Nutzung eines handelsüblichen Tablet-PCs oder eines Smartphones, das von einem für die erfindungsgemäße Vorrichtung vorgesehenen Programm gesteuert wird. Damit liegt eine multifunktionale Bedieneinheit vor, die über einen berührungsempfindlichen Bildschirm sowie wenigstens eine Einrichtung zur drahtlosen Kommunikation verfügt.

Weitere Einzelheiten, Merkmale und Vorteile der Erfindung ergeben sich aus der nachfolgenden Beschreibung eines Ausführungsbeispiels mit Bezugnahme auf die zugehörigen Zeichnungen. Es zeigen:
Fig. 1: schematisch eine Prinzipdarstellung einer Ausführungsform eines Anzugs einer erfindungsgemäßen Vorrichtung zur komplexen Elektromyostimulation;
Fig. 2: schematisch eine Prinzipdarstellung einer Ausführungsform einer Kautschukelektrode einer erfindungsgemäßen Vorrichtung zur komplexen Elektromyostimulation;
Fig. 3: schematisch eine Prinzipdarstellung einer Ausführungsform einer erfindungsgemäßen Vorrichtung zur komplexen Elektromyostimulation;
Fig. 4: schematisch eine Prinzipdarstellung einer Ausführungsform einer Stimulationseinheit und
Fig. 5: schematisch eine Prinzipdarstellung einer weiteren Ausführungsform einer erfindungsgemäßen Vorrichtung zur komplexen Elektromyostimulation.

Fig. 1 zeigt schematisch eine Darstellung einer Ausführungsform eines Anzugs 2 zum Einsatz in einer erfindungsgemäßen Vorrichtung zur komplexen Elektromyostimulation 1. Der Anzug 2 ist flachgestrickt und weist an verschiedenen vorgesehenen Stellen auf der Vorderseite und der Rückseite Kontaktelektroden 6 auf. Diese bestehen im Wesentlichen aus einer unter Verwendung elektrisch leitfähiger Fäden flachgestrickten Intarsie 3, eingearbeitet in das Flachgestrick 5 des Anzugs, und weisen zur Haut des Nutzers hin eine Implantierung 4 auf. Die Implantierung 4, bevorzugt aus Kautschuk bestehend, ist in der Weise mit der Intarsie 3 verbunden, dass eine mechanisch feste und elektrisch sicher leitfähige Verbindung entsteht. Die Herstellung des Anzugs 2 im Flachstrickverfahren ermöglicht eine optimale Anpassung an die Körperform.

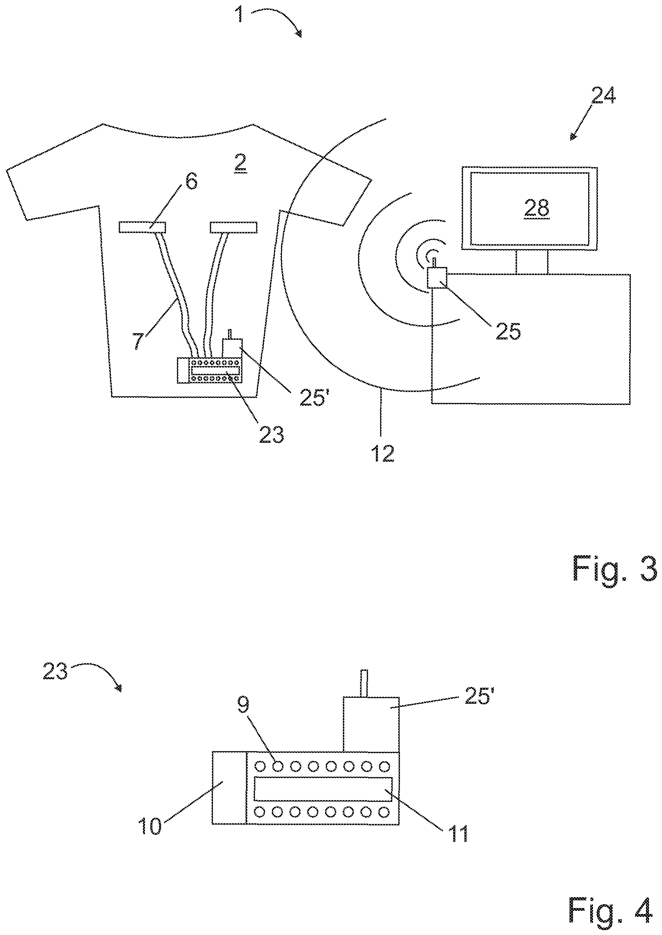

Die elektrische Anbindung der Kontaktelektroden 6 erfolgt mittels elektrischer Leitungen 7, die in der bevorzugten Ausführungsform ebenfalls aus elektrisch leitfähigen Fäden flachgestrickt sind und darüber hinaus in eine Tunnelröhre 8 eingestrickt sind. Diese Tunnelröhre 8 schützt die elektrische Leitung 7 vor mechanischer Beschädigung, dient als Isolierung und zugleich einem vorteilhaften optischen Eindruck des Anzugs 2, zum Beispiel bei der Verwendung als Zierelement. Die elektrischen Leitungen 7 sind jeweils angeschlossen an einen metallisierten Druckknopf 9, der an der Außenseite des Anzug 2 angebracht ist und der Verbindung mit der Stimulationseinheit 23 (vergleiche Fig. 3, 4 oder 5) dient.

Fig. 2 zeigt schematisch eine geschnittene Darstellung einer Ausführungsform einer Kontaktelektrode 6, 26 einschließlich der elektrischen Anbindung, die in das Flachgestrick 5 eines erfindungsgemäßen Anzuges eingebracht ist. Eine flachgestrickten Intarsie 3, die unter Verwendung elektrisch leitfähiger Fäden hergestellt ist, ist in das den Anzug bildende Flachgestrick 5 eingearbeitet. Im vorliegenden Ausführungsbeispiel durchdringt die Intarsie 3 das Flachgestrick 5 in voller Stärke, alternativ ist auch ein nur teilweises Eindringen vorgesehen. Weiterhin wird in dem Flachgestrick 5 die ebenfalls bevorzugt gestrickte, aber auch als metallischer Leiter oder auf andere geeignete Weise ausführbare elektrische Leitung 7 herangeführt, die ihrerseits in einer schützenden und isolierenden Tunnelröhre 8 verläuft. Auch die Tunnelröhre 8 wird bevorzugt im Flachstrickverfahren hergestellt. Dadurch kann der Anzug bzw. ein Anzugteil in einem Arbeitsgang komplett gefertigt werden, soweit es die textiltechnische Ausführung betrifft.

Auf die Intarsie 3 wird ein elektrisch leitfähiges Material, bevorzugt Kautschuk, in der Weise aufgebracht, dass eine intensive Verbindung zwischen der Intarsie 3 und der Implantierung 4 entsteht, bei der sich Intarsie 3 und Implantierung 4 teilweise durchdringen. Dies sichert die hohe mechanische Festigkeit gegen Ablösen, eine sehr gute elektrische Verbindung zwischen Intarsie 3 und Implantierung 4 sowie übereinstimmende Dehnungseigenschaften zwischen Flachgestrick 5, Intarsie 3 und Implantierung 4.

Die Oberfläche der Implantierung 4 ist zur Anlage auf der Haut vorgesehen, sorgt damit für eine großflächige Übertragung der elektrischen Reize auf die Haut und ruft darüber hinaus eine leichte lokale Eigenbefeuchtung der Kontaktstelle durch die Haut hervor, was den Übergang des elektrischen Stroms von der Kontaktelektrode 6, 26 zur Haut noch weiter verbessert.

Fig. 3 zeigt schematisch eine Prinzipdarstellung einer Ausführungsform einer erfindungsgemäßen Vorrichtung 1 zur komplexen Elektromyostimulation mit dem Anzug 2, an dem die Stimulationseinheit 23 angeordnet ist. Weitere Hauptkomponente ist die Bedieneinheit 24, die als Teil der Kommunikationseinheit den Bluetooth-Sender 25 aufweist. Dieser kommuniziert mit dem Bluetooth-Empfänger 25, verbunden mit der Stimulationseinheit 23.

Die Stimulationseinheit 23 ist über elektrische Leitungen 7 mit den Kontaktelektroden 6, die an dem oder in dem textilen Flächengebilde des Anzugs 2 angeordnet sind, verbunden. Hierdurch werden die Modulationsimpulse, elektrische Ströme, zu den Kontaktelektroden 6 übertragen und dort, bedingt durch den Hautkontakt, in den Körper des Nutzers auf die Muskulatur übertragen. Hierzu sind die Kontaktelektroden 6 so ausgebildet, dass ein geringer Übergangswiderstand zur Haut auf der Gesamtfläche der Kontaktelektroden 6 gewährleistet ist. Damit wird die vorgesehene Stimulationsdosis übertragen, ohne jedoch die Nerven der Haut zu reizen und ein als unangenehm empfundenes Stechen hervorzurufen. Dieses wird in der bevorzugten Ausführungsform durch die zuvor beschriebene Implantierung mit elektrisch leitendem Kautschuk erreicht. Die elektrische Verbindung erfolgt bevorzugt über die textilbasierte Leitungen.

Die Bedieneinheit 24 weist den berührungsempfindlichen Bildschirm 28 auf. Auf diesem ist eine bevorzugt auf den Nutzer bezogene, optimierte Bedienoberfläche darstellbar. Gleichfalls ist eine kontextabhängige Veränderung möglich. Die von Anzug 2 und Stimulationseinheit 23 entfernt angeordnete Bedieneinheit 24 ermöglicht im Vergleich zu einer tragbaren Bedieneinheit diesbezüglich weitaus größere Möglichkeiten, ganz abgesehen von den programmtechnischen Möglichkeiten, die beispielsweise ein in die Bedieneinheit integrierter Computer hinsichtlich Rechnerleistung oder Anbindung an ein Netzwerk mit sich bringt.

Die drahtlose Übertragung zwischen dem Bluetooth-Sender 25 und dem Bluetooth-Empfänger 25' ist durch das stilisierte Bluetooth-Signal 12 dargestellt.

Fig. 4 zeigt schematisch eine Prinzipdarstellung einer Ausführungsform einer Stimulationseinheit zur mechanischen und zugleich elektrischen Verbindung mit einem Anzug oder einer Strumpfhose gemäß Fig. 3, beispielhaft dargestellt in der bevorzugten Ausführungsform mit metallisierten Druckknöpfen 9, hier insgesamt 16 Stück. Die Gegenstücke der Druckknöpfe 9 befinden sich an dem Anzug. Zur Verbindung der Kontakte des Anzuges mit den als Druckknöpfe 9 ausgearbeiteten Gegenkontakten an der Stimulationseinheit 23 werden die Druckknöpfe 9 ineinander gedrückt. Die Stimulationseinheit 23 weist weiterhin den Akkumulator 10 auf, der der Stromversorgung dient und bevorzugt zur externen Aufladung abnehmbar ausgeführt ist. Weiterhin weist die Stimulationseinheit 23 eine Steuerplatine 11 auf, die die von dem Bluetooth-Empfänger 25' empfangenen Signale in der Weise umsetzt, dass Stimulationsströme, elektrische Impulse, über die Druckknöpfe 9 fließen. Alternativ werden auch einfache programmtechnische Schritte in der Steuerplatine 11 ausgeführt, um die Kommunikationseinheit zu entlasten.

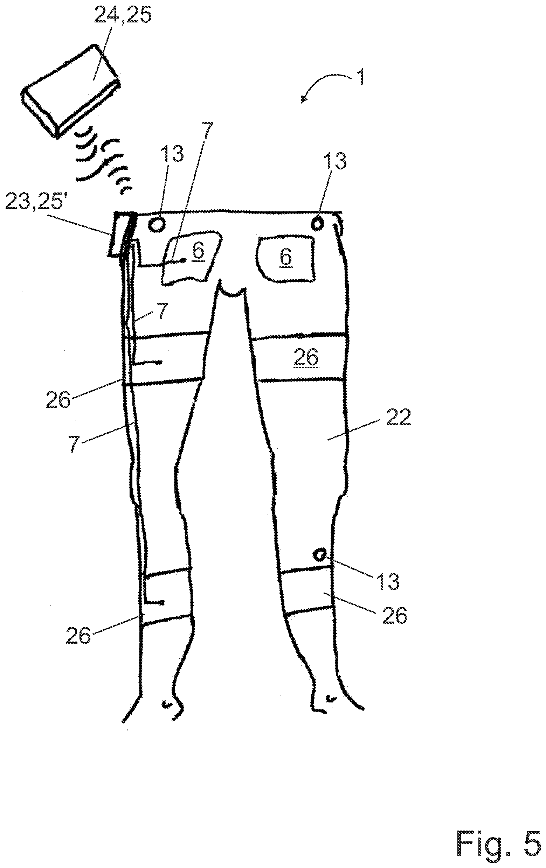

Fig. 5 zeigt schematisch eine Prinzipdarstellung einer weiteren Ausführungsform einer erfindungsgemäßen Vorrichtung zur komplexen Elektromyostimulation als elektrische Strumpfhose 22. Es handelt sich dabei um eine Strumpfhose 22, in die elektrisch leitende Flächen, z.B. Intarsien oder Flachgestrick, als Kontaktelektroden 6, 26 eingearbeitet sind, wobei zirkumferentielle Kontaktelektroden 26 die Oberschenkel-und/ oder die Unterschenkelmuskulatur umschließen. Kontaktelektroden 6 liegen eben der Gesäßmuskulatur auf. Die Strumpfhose kann wahlweise den gesamten Bein- und Fußbereich umschließen oder nur so weit reichen, dass auch die zirkumferentiellen Kontaktelektroden 26 an den Unterschenkeln durch die Strumpfhose 22 in ihrer Position fixiert werden.

Die elektrische Verbindung dieser Kontaktelektroden 6, 26 zu der Stimulationseinheit 23 erfolgt durch in das Textil integrierte elektrische Leitungen 7. Die Stimulationseinheit ist bevorzugt im Hüftbereich in das Textil eingelassen oder auf andere Weise dort permanent oder temporär angebracht. Im Textil integriert sind ferner Sensoren 13, bevorzugt als Signalaufnehmer zur Registrierung des EKG. Bevorzugte Orte für die Anbringung von Sensoren 13 sind die rechte Hüfte, die linke Hüfte und/ oder der Unterschenkel. Durch die Verarbeitung des EKG in einer in der Stimulationseinheit 23 oder der Bedieneinheit 24 integrierten Verarbeitungseinheit sind die Stimulationsströme beeinflussbar. Somit ist insbesondere wahlweise ein herzsynchroner oder ein asynchroner Stimulationsmodus ansteuerbar. Die Kommunikation zwischen Bedieneinheit 24 und Stimulationseinheit 23 erfolgt bevorzugt drahtlos über die Kommunikationseinheit 25, 25'.

Die als Stimulationsschicht ausgebildeten textilbasierten elektrisch leitfähigen Flächen der Kontaktelektroden 6, 26 sind mit einer aus Kautschuk bestehenden Implantation, bevorzugt aus einem ionisierten Polymer vulkanisiert. Die Stimulationsschicht ist in der Lage, den Strom ohne zusätzliche Befeuchtung auf die menschliche Haut zu transferieren.

Die Stimulationseinheit 23 wird durch eine Bedieneinheit 24 angesteuert. Bevorzugt kommt hierzu ein Tablet-PC oder ein Smartphone zum Einsatz, das durch eine spezielle Software angesteuert wird. Durch die bei solchen Geräten standardmäßig vorhandene Möglichkeit zur drahtlosen Signalübertragung durch eine integrierte Kommunikationseinheit 25, 25' entfallen sämtliche einer sicheren und einfachen Bedienung hinderlichen Verkabelungen. Der Tragekomfort erlaubt damit die Anwendung im Alltag, eine Hilfsperson ist nicht notwendig.

Weitere Vorteile der erfindungsgemäßen Vorrichtung liegen darin, bereits anerkannte und validierte Therapieoptionen derart nutzerfreundlich zu gestalten, dass diese von der Zielgruppe akzeptiert werden und ohne fremde Hilfe im häuslichen Milieu angewandt werden können. Somit kommt es zu einer niedrigeren Re-Hospitalisierungsrate der Zielgruppe, insbesondere herzinsuffiziente Patienten, und zu einer positiven Beeinflussung der Grunderkrankung.

### Bezugszeichenliste

- 1: Vorrichtung zur komplexen Elektromyostimulation
- 2: Anzug
- 3: Intarsie
- 4: Implantierung
- 5: Flachgestrick
- 6: Kontaktelektrode
- 7: elektrische Leitung
- 8: Tunnelröhre
- 9: metallisierter Druckknopf
- 10: Akkumulator
- 11: Steuerplatine
- 12: Bluetooth-Signal
- 13: Sensor
- 23: Stimulationseinheit
- 22: Strumpfhose
- 24: Bedieneinheit
- 25: Kommunikationseinheit, Bluetooth-Sender
- 25': Kommunikationseinheit, Bluetooth-Empfänger
- 26: zirkumferentielle Kontaktelektrode
- 28: berührungsempfindlicher Bildschirm

## Patentansprüche

1. Vorrichtung zur muskulären Stimulation von Rumpf- und/ oder Beinmuskulatur durch Elektromyostimulation, umfassend einen wenigstens eine integrierte textilbasierte Kontaktelektrode (6, 26) und wenigstens eine elektrische Leitung (7) aufweisenden, an einem Körper anliegenden, ein- oder mehrteiligen, aus einem elastischen textilen Flächengebilde bestehenden Anzug (2), wobei das Flächengebilde als wenigstens ein Flachgestrick (5) ausgeführt ist, wobei die Kontaktelektrode (6, 26) als ein zumindest einschichtiges, als Intarsie (3) ausgebildetes Flachgestrick zumindest teilweise aus elektrisch leitfähigem Faden ausgeführt ist, **dadurch gekennzeichnet, dass** in dem textilen Flächengebilde die elektrische Leitung (7) in einer gestrickten Tunnelröhre (8) angeordnet ist, wobei die Kontaktelektrode (6, 26) mittels der Leitung (7) elektrisch konnektierbar ist und das als Intarsie (3) ausgebildete Flachgestrick zumindest an der dem Körper zugewandten Seite eine elastische, elektrisch leitende Implantierung (4) oder einen auf dem Flachgestrick (5) angeordneten elektrisch leitenden Kautschuk aufweist.

2. Vorrichtung nach Anspruch 1, wobei das Flachgestrick (5) des Anzugs (2) einflächig ausgeführt ist.

3. Vorrichtung nach Anspruch 1 oder 2, wobei die elektrische Leitung (7) zumindest teilweise aus elektrisch leitfähigem Faden nach einem textiltechnischen Verfahren gefertigt ist.

4. Vorrichtung nach einem der vorherigen Ansprüche, wobei die elektrische Leitung (7) gestrickt ist.

5. Vorrichtung nach einem der vorherigen Ansprüche, wobei das Flachgestrick (5), die Tunnelröhre (8) und die elektrische Leitung (7) miteinander ausgebildet sind.

6. Vorrichtung nach einem der vorherigen Ansprüche, wobei der elektrisch leitfähige Faden von Intarsie (3) oder Leitung (7) aus einem Kohlenstoffpolymer, aus einem Metall oder einer Metalllegierung besteht oder eine Kohlenstoff- oder Metallbeschichtung aufweist.

7. Vorrichtung nach einem der vorherigen Ansprüche, wobei der Anzug (2) aus mehreren Flachgestricken (5) besteht, deren elektrische Leitungen (7) beim Zusammenfügen zu dem Anzug (2) zur elektrisch leitenden Verbindung kontaktiert werden.

8. Vorrichtung nach einem der vorherigen Ansprüche, wobei das zumindest einschichtige, als Intarsie (3) ausgebildete Flachgestrick den vollen Querschnitt des Flachgestricks (5) durchdringt oder ein- oder beidseitig in die Oberfläche des Flachgestricks (5) eingearbeitet ist.

9. Vorrichtung nach einem der vorherigen Ansprüche, wobei als elektrisch leitende Implantierung (4) ein elektrisch leitender Kautschuk vorgesehen ist, der im Bereich der Oberfläche des als Intarsie (3) ausgebildeten Flachgestricks in der Weise eingebettet ist, dass das Flachgestrick zumindest teilweise überdeckt wird.

10. Vorrichtung nach einem der vorherigen Ansprüche, wobei der Anzug (2) wenigstens teilweise als Strumpfhose (22) ausgebildet ist und wenigstens eine zirkumferentielle Kontaktelektrode (26) im Bereich von Ober- oder Unterschenkel aufweist.

11. Vorrichtung nach einem der vorherigen Ansprüche, wobei eine Stimulationseinheit (23), eine Bedieneinheit (24) sowie eine die Bedieneinheit (24) und die Stimulationseinheit (23) zur Signalübertragung verbindende Kommunikationseinheit (25, 25') vorgesehen sind.

12. Vorrichtung nach Anspruch 11, wobei die Bedieneinheit (24) als grafische Nutzerschnittstelle, aufweisend einen berührungsempfindlichen Bildschirm (28), und zur Steuerung der Aktivität der Kontaktelektroden (6, 26) ausgebildet ist und wobei die Bedieneinheit (24) von dem Anzug (2) beabstandet ist.

13. Vorrichtung nach einem der Ansprüche 11 oder 12, wobei in der Bedieneinheit (24) abrufbare Programme zur Steuerung der Aktivität wenigstens einer Kontaktelektrode (6, 26) abgespeichert werden und die Stimulationseinheit (23) zur steuerbaren Energieversorgung der Kontaktelektroden (6, 26) über die elektrischen Leitungen (7) zwischen der Stimulationseinheit und den Kontaktelektroden auf Grundlage der Programme zur Steuerung der Aktivität ausgeführt ist.

14. Vorrichtung nach einem der Ansprüche 11 bis 13, wobei die Stimulationseinheit (23) abnehmbar mit dem Anzug (2) verbunden ist.

15. Vorrichtung nach einem der Ansprüche 11 bis 14, wobei die Kommunikationseinheit (25, 25') zur drahtlosen Signalübertragung ausgebildet ist.

## Claims

1. A device for muscle stimulation of the trunk and/or leg musculature by electromyostimulation, comprising a one-part or multi-part suit (2) made of an elastic textile fabric and including at least one integrated textile-based contact electrode (6, 26) and at least one electric line (7) and bearing on a body, the textile fabric being implemented as at least one flat-knit (5), the contact electrode (6, 26) being implemented as an at least one-layered flat-knit formed as an intarsia (3) and at least partially made of electrically conductive yarn, **characterized in that**, in the textile fabric, the electric line (7) is arranged in a knitted tunnel tube (8), the contact electrode (6, 26) being electrically connectable by means of the line (7), and the flat-knit formed as an intarsia (3) including, at least on the side facing the body, an elastic, electrically conducting implantation (4) or an electrically conducting natural rubber arranged on the flat-knit (5).

2. The device according to claim 1, wherein the flat-knit (5) of the suit (2) is implemented as single-faced.

3. The device according to claim 1 or 2, wherein the electric line (7) is at least partially manufactured from electrically conductive yarn according to a textile engineering process.

4. The device according to any one of the preceding claims, wherein the electric line (7) is knitted.

5. The device according to any one of the preceding claims, wherein the flat-knit (5), the tunnel tube (8) and the electric line (7) are formed together.

6. The device according to any one of the preceding claims, wherein the electrically conductive yarn of the intarsia (3) or line (7) consists of a carbon polymer, a metal or a metal alloy, or includes a carbon or metal coating.

7. The device according to any one of the preceding claims, wherein the suit (2) consists of multiple flat-knits (5), the electric lines (7) of which are contacted for electrically conducting connection when being joined together to form the suit (2).

8. The device according to any one of the preceding claims, wherein the at least one-layered flat-knit formed as an intarsia (3) penetrates the entire cross-section of the flat-knit (5) or is incorporated in the surface of the flat-knit (5) on one or both sides.

9. The device according to any one of the preceding claims, wherein an electrically conducting natural rubber, which is embedded in the region of the surface of the flat-knit formed as an intarsia (3) such that the flat-knit is at least partially covered, is provided as electrically conducting implantation (4).

10. The device according to any one of the preceding claims, wherein the suit (2) is at least partially formed as tights (22) and includes at least one circumferential contact electrode (26) in the upper or lower leg region.

11. The device according to any one of the preceding claims, wherein a stimulation unit (23), an operating unit (24) and a communication unit (25, 25') connecting the operating unit (24) and the stimulation unit (23) for signal transmission are provided.

12. The device according to claim 11, wherein the operating unit (24) is designed as a graphical user interface including a touchscreen (28) and is configured to control the activity of the contact electrodes (6, 26), and wherein the operating unit (24) is remote from the suit (2).

13. The device according to any one of claims 11 or 12, wherein selectable programs for controlling the activity of at least one contact electrode (6, 26) are stored in the operating unit (24), and the stimulation unit (23) is configured to supply the contact electrodes (6, 26) with energy via the electric lines (7) between the stimulation unit and the contact electrodes in a controlled manner based on the programs for controlling the activity.

14. The device according to any one of claims 11 to 13, wherein the stimulation unit (23) is removably connected to the suit (2).

15. The device according to any one of claims 11 to 14, wherein the communication unit (25, 25') is configured for wireless signal transmission.

## Revendications

1. Dispositif de stimulation musculaire de la musculature du tronc et/ou de la jambe par électromyostimulation, comprenant un costume (2) présentant au moins une électrode de contact intégrée (6, 26) à base de textile et au moins un câble électrique (7), adjacent à un corps, en une ou plusieurs parties, composé d'une structure plane textile élastique, dans lequel la structure plane est réalisée en tant qu'au moins un tricot plat (5), dans lequel l'électrode de contact (6, 26) est réalisée en tant qu'un tricot plat au moins à une couche, réalisé en tant qu'incrustation (3), au moins partiellement en fil électriquement conducteur, **caractérisé en ce que** le câble électrique (7) dans la structure plane textile est disposé dans un tube de tunnel tricoté (8), dans lequel l'électrode de contact (6, 26) peut être connectée électriquement au moyen du câble (7) et le tricot plat réalisé en tant qu'incrustation (3) présente au moins sur le côté tourné vers le corps une implantation élastique (4), électriquement conductrice, ou un caoutchouc électriquement conducteur, disposé sur le tricot plat (5).

2. Dispositif selon la revendication 1, dans lequel le tricot plat (5) du costume (2) est réalisé de manière monoface.

3. Dispositif selon la revendication 1 ou 2, dans lequel le câble électrique (7) est fabriqué au moins partiellement en fil électriquement conducteur selon un procédé technique du textile.

4. Dispositif selon une des revendications précédentes, dans lequel le câble électrique (7) est tricoté.

5. Dispositif selon une des revendications précédentes, dans lequel le tricot plat (5), le tube de tunnel (8) et le câble électrique (7) sont réalisés les uns avec les autres.

6. Dispositif selon une des revendications précédentes, dans lequel le fil électriquement conducteur de l'incrustation (3) ou le câble (7) se compose d'un polymère de carbone, d'un métal ou d'un alliage métallique ou présente un enrobage de carbone ou métallique.

7. Dispositif selon une des revendications précédentes, dans lequel le costume (2) se compose de plusieurs tricots plats (5) dont les câbles électriques (7) sont soumis à un contact électrique pour la connexion électriquement conductrice lors de l'assemblage au costume (2).

8. Dispositif selon une des revendications précédentes, dans lequel le tricot plat au moins à une couche, réalisé en tant qu'incrustation (3), perce la section transversale entière du tricot plat (5) ou est incorporé d'un côté ou des deux côtés dans la surface du tricot plat (5).

9. Dispositif selon une des revendications précédentes, dans lequel un caoutchouc électriquement conducteur est prévu en tant qu'implantation électriquement conductrice (4), lequel est encastré dans la région de la surface du tricot plat réalisé en tant qu'incrustation (3) de telle sorte que le tricot plat est au moins partiellement recouvert.

10. Dispositif selon une des revendications précédentes, dans lequel le costume (2) est réalisé au moins partiellement en tant que collant (22) et présente au moins une électrode de contact circonférentielle (26) dans la région de la cuisse ou du bas de la jambe.

11. Dispositif selon une des revendications précédentes, dans lequel un module de stimulation (23), un module de commande (24) ainsi qu'un module de communication (25, 25') connectant le module de commande (24) et le module de stimulation (23) pour la transmission de signal sont prévus.

12. Dispositif selon la revendication 11, dans lequel le module de commande (24) est réalisé en tant qu'interface utilisateur graphique présentant un écran tactile (28) et pour la commande de l'activité des électrodes de contact (6, 26), et dans lequel le module de commande (24) est espacé du costume (2).

13. Dispositif selon une des revendications 11 ou 12, dans lequel des programmes pouvant être appelés pour la commande de l'activité d'au moins une électrode de contact (6, 26) sont mémorisés dans le module de commande (24) et le module de stimulation (23) est réalisé pour l'alimentation électrique pouvant être commandée des électrodes de contact (6, 26) par le biais des câbles électriques (7) entre le module de stimulation et les électrodes de contact en fonction des programmes pour la commande de l'activité.

14. Dispositif selon une des revendications 11 à 13, dans lequel le module de stimulation (23) est connecté de manière amovible au costume (2).

15. Dispositif selon une des revendications 11 à 14, dans lequel le module de communication (25, 25') est réalisé pour la transmission de signal sans fil.
